(19) Europäisches Patentamt
European Patent Office
Office européen des brevets

(11) EP 2 512 420 B1

(12) # FASCICULE DE BREVET EUROPEEN

(45) Date de publication et mention de la délivrance du brevet:
**04.09.2019 Bulletin 2019/36**

(21) Numéro de dépôt: **10792933.3**

(22) Date de dépôt: **17.12.2010**

(51) Int Cl.:
***A61K 31/215*** (2006.01) ***A61Q 19/00*** (2006.01)
***A61K 8/37*** (2006.01)

(86) Numéro de dépôt international:
**PCT/EP2010/070028**

(87) Numéro de publication internationale:
**WO 2011/073370 (23.06.2011 Gazette 2011/25)**

(54) **UTILISATION DU 2,3-DIHYDROXYPROPYL DODÉCANOATE POUR LE TRAITEMENT DE LA SÉBORRHÉE**

VERWENDUNG VON 2,3-DIHYDROXYPROPYL-DODECANOAT ZUR BEHANDLUNG VON SEBORRHÖ

USE OF 2,3-DIHYDROXYPROPYL DODECANOATE FOR TREATING SEBORRHEA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.12.2009 FR 0959165**

(43) Date de publication de la demande:
**24.10.2012 Bulletin 2012/43**

(73) Titulaire: **Pierre Fabre Dermo-Cosmétique
92100 Boulogne-Billancourt (FR)**

(72) Inventeurs:
- **DECHELETTE, Corinne
F-81800 Rabastens (FR)**
- **REDOULES, Daniel
F-31300 Toulouse (FR)**

(74) Mandataire: **Regimbeau
20, rue de Chazelles
75847 Paris Cedex 17 (FR)**

(56) Documents cités:
**FR-A1- 2 867 973 US-A- 4 921 694
US-A- 6 110 908 US-A1- 2009 203 628**

- **NIEDERPRUEM H-J ET AL: "INHIBITION OF STEROID 5ALPHA-REDUCTASE ACTIVITY BY ALIPHATIC FATTY ACIDS CANDIDATES FOR CHEMOPREVENTION OF PROSTATE CANCER", ANNALS OF THE NEW YORK ACADEMY OF SCIENCES, NEW YORK ACADEMY OF SCIENCES, NEW YORK, NY, US LNKD-DOI:10.1111/J.1749-6632.1995.TB12127.X, vol. 768, 1 janvier 1995 (1995-01-01), pages 227-230, XP000949291, ISSN: 0077-8923**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 512 420 B1

## Description

La présente invention concerne le 2,3-dihydroxypropyl dodécanoate pour son utilisation en tant que seul principe actif pour le traitement de la séborrhée. Il peut s'agir de la séborrhée de la peau ou du cuir chevelu.
L'invention concerne également les compositions cosmétiques comprenant le 2,3-dihydroxypropyl dodécanoate en tant que seul principe actif pour son utilisation dans le traitement de la séborrhée.
Le sébum est la sécrétion par les glandes sébacées de la peau d'un film lipidique qui sert à la protéger et, mélangée à la sueur, protège la peau du dessèchement.
Le sébum permet l'imperméabilisation de la peau, participe au développement de la structure épidermique.
Il la protège des microbes en l'acidifiant (présence d'acide lactique et d'acides gras) et assure une certaine imperméabilité. Il permet à la peau d'être souple et participe au développement de l'épiderme.
Il est également présent sur les poils et les cheveux.
Le sébum est produit par les glandes sébacées.
Globalement, l'homme possède 2 000 000 de glandes sébacées annexées à 6 000 000 de poils et cheveux.
La répartition des glandes sébacées est non uniforme. La densité des glandes sébacées atteint 300 à 900 glandes sébacées/$cm^2$ au niveau du visage et du cuir chevelu, et cette densité est de l'ordre de 100 glandes sébacées/$cm^2$ dans la partie haute du thorax et du dos.
La glande sébacée a un mode de sécrétion holocrine c'est-à-dire élimination totale de la cellule. Sa portion sécrétoire est de type alvéolaire.
La séborrhée correspond à une production excessive de sébum par les glandes sébacées.
La séborrhée peut avoir différentes causes :

- Le système nerveux : stress émotionnel, tension nerveuse exacerbant la fonction sébacée,
- Fatigue,
- Alimentation mal équilibrée,
- Certains médicaments (psychotropes),
- Soins cosmétiques inadaptés « décapant » la peau et/ou le cuir chevelu, et provoquant une séborrhée réactionnelle,
- La cause principale est de type hormonal : La glande sébacée est hormono-dépendante et son activité est influencée par les androgènes. Les androgènes ne sont actifs que sous l'influence de l'enzyme 5-$\alpha$ réductase qui assure la métabolisation des androgènes au niveau la glande sébacée ce qui induit la production de sébum. Une hyperactivation de l'enzyme 5-$\alpha$ réductase provoque la séborrhée.

Le siège des manifestations de la séborrhée est la région médio-faciale (front, nez menton) où les glandes sébacées sont les plus nombreuses et les plus volumineuses. La séborrhée se manifeste également au niveau du cuir chevelu où elle prédomine dans les régions frontale, fronto-temporale et au sommet du crâne.
La séborrhée occasionne des désagréments d'ordre esthétique. En ce qui concerne la peau, elle présente un aspect luisant, le teint est terne et les orifices pilo-sébacés sont dilatés. De plus, le maquillage ne présente pas une bonne tenue sur ce type de peaux dites grasses.
Le document US2009203628 décrit une méthode de traitement de la séborrhée en utilisant des composés inhibiteurs de 5-alpha réductase et d'agents kératolytiques.
Pour la séborrhée du cuir chevelu, les cheveux présentent un aspect gras et terne et ils sont difficiles à coiffer. Quand la séborrhée est intense, elle est dite huileuse, fluente et elle peut être associée à une odeur rance.
Pour traiter la séborrhée et résoudre les désagréments d'ordre esthétique liés à la séborrhée, il a été mis en évidence de façon surprenante que l'utilisation du 2,3-dihydroxypropyl dodécanoate permet d'inhiber l'activité de l'enzyme 5-$\alpha$ réductase et donc de réduire la sécrétion de sébum.

1) Le 2,3-dihydroxypropyl dodécanoate :

Le 2,3-dihydroxypropyl dodécanoate (Formule 1), également appelé le glycéryl laurate ou glycéryl monolaurate, est un ester formé à partir de l'acide laurique et le glycérol de formule chimique $C_{15}H_{30}O_4$. Il est usuellement utilisé en tant qu'émulsionnant comme par exemple dans les produits d'entretien ménager mais également dans certains produits alimentaires.

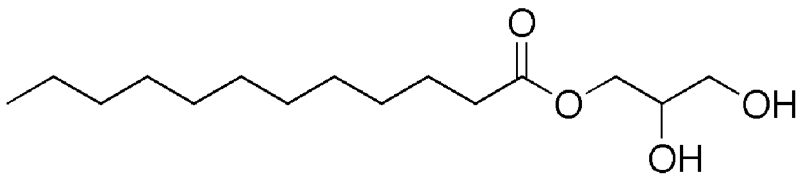

Formule 1 : 2,3-dihydroxypropyl dodécanoate

2) L'enzyme 5-α réductase

Il existe deux isozymes de l'enzyme 5-α réductase : la 5-α réductase 1 et la 5-α réductase 2. Le type 1 est exprimée essentiellement dans la peau , localisée dans la glande sébacée et la papille dermique et agit à pH 7, tandis que le type 2 est exprimée dans la prostate et agit à pH5 (Luu-The et al, Characterization, Expression and Immunohistochemical localization of 5-alpha reductase in human skin, The Journal of Investigate Dermatology, p221-226, 1994).

L'enzyme 5-α réductase est une enzyme impliquée dans le métabolisme des stéroïdes (Luu-The et al, Characterization, Expression and Immunohistochemical localization of 5-alpha reductase in human skin, The Journal of Investigate Dermatology, p221-226, 1994).

Plus spécifiquement cette enzyme réduit la liaison Δ4,5 de la testostérone (Formule 2) avec production de la dihydrotestostérone (androstanolone) (DHT) (Formule 3) qui est également une hormone androgène.

La glande sébacée est hormono-dépendante, et son fonctionnement est lié à la testostérone. La testostérone est fabriquée par les testicules, les ovaires (androstène dione) et les glandes surrénales (déhydroépiandrostérone). Dans les cellules cibles, la testostérone est transformée en DHT active par l'enzyme 5-α réductase. Après s'être combinée à un récepteur cytosolique, la DHT se fixe sur un récepteur nucléaire et elle induit la synthèse de protéines responsables de la production de sébum.

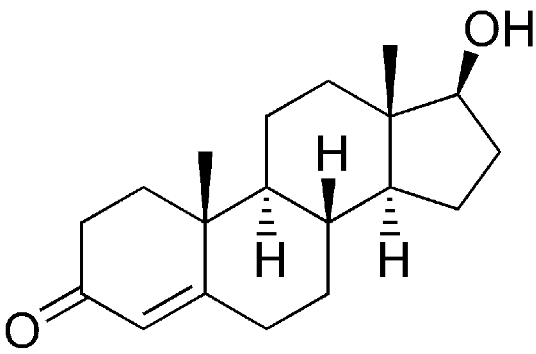

Formule 2 : Testostérone

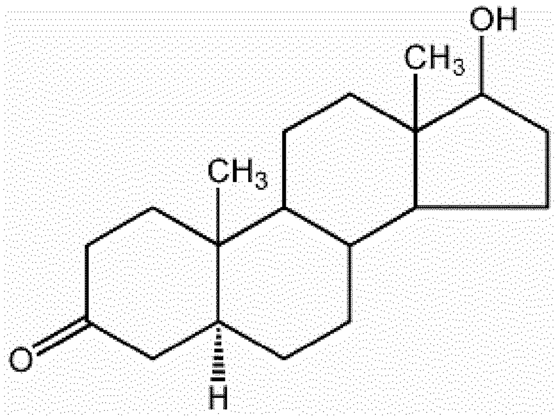

Formule 3 : Dihydrotestostérone (DHT)

La séborrhée est liée à une hyperactivité de l'enzyme 5-α réductase associée à une augmentation du nombre de récepteurs cytosoliques.

Le fait d'inhiber l'activité de l'enzyme 5-α réductase permet donc de diminuer la métabolisation des androgènes et donc la production de sébum.

3) Résultats biologiques : Inhibition de l'activité de la 5-α réductase par le 2,3-dihydroxypropyl dodécanoate :

Protocole :

Modèle biologique :

- Type cellulaire : Fibroblastes humains normaux (NHDF = normal human dermal fibroblasts).
- Conditions de culture : 37°C, 5% $CO_2$.
- Milieu de culture : DMEM supplémenté par 2 mM de L-glutamine, 50 U/ml de Pénicilline, 50μg/ml de Streptomycine, 10% de sérum de veau foetal.
- Milieu de culture de l'essai : DMEM supplémenté avec 2 mM de L-glutamine, 50 U/ml de Pénicilline, 50μg/ml de Streptomycine et 1% de sérum de veau foetal.
- Testostérone radiomarquée : [4-14C]testostérone (Amersham). La testostérone radiomarquée a été dissoute dans de l'éthanol et diluée dans le milieu de culture de l'essai.

Composé testé :

- Composé testé : le 2,3-dihydroxypropyl dodécanoate.
- Témoin positif : le finastéride.
- Le finastéride est bien connu pour inhiber l'activité de la 5-α réductase et ainsi empêcher la métabolisation de la testostérone en DHT.
- Contrôle: l'éthanol, qui n'a pas d'activité d'inhibition de la 5-α réductase.

Culture cellulaire et traitement :

Les fibroblastes ont été cultivés jusqu'à confluence. Le milieu de culture a ensuite été retiré et remplacé par le milieu de culture de l'essai contenant ou pas le composé testé, le témoin positif ou le contrôle et les cellules ont été pré-incubées pendant 24 heures. Après incubation, les cellules ont été traitées avec le composé testé ou le témoin négatif et la testostérone radiomarquée a été ajoutée. Les cellules ont ensuite été incubées pendant 24 heures. Toutes les expériences ont été réalisées trois fois.

Extractions et analyses :

Les stéroïdes ont été extraits des surnageants avec deux volumes de chloroforme/méthanol et séchés. Les différentes entités moléculaires (les métabolites de la testostérone) ont été séparées par chromatographie sur couche mince sur plaques de silice (RE/silice, Whatman) dans un mélange de solvants comprenant le dichlorométhane, l'éthylacétate et le méthanol.

Les plaques ont été autoradiographiées et les métabolites de la testostérone ont été quantifiés en utilisant un phosphorImager qui est un détecteur de rayonnement béta et un logiciel spécifique (Packard Instrument).

La diminution de la quantité de DHT radiomarquée détectée correspond à une inhibition de l'activité de l'enzyme 5-α réductase.

Le traitement des données :

- Les données brutes ont été analysées avec un logiciel Microsoft Excel ®.
- Calcul de l'erreur type de la moyenne :

$$Erreur\ type\ de\ la\ moyenne = \frac{Ecart - type}{\sqrt{(n)}}$$

- Calcul de la Viabilité

$$Viabilit\acute{e} = \frac{Densit\acute{e}\ optique\ de\ l'\acute{e}chantillon}{Densit\acute{e}\ optique\ du\ contr\hat{o}le} \times 100$$

Les résultats sont exprimés sur la figure 1 annexée qui illustre les effets du 2,3-dihydroxypropoyl dodécanoate sur la production de DHT par les fibroblastes humains.

On observe que le contrôle l'éthanol, n'a aucun effet sur la métabolisation de la testostérone et donc n'inhibe

pas l'activité de la 5-α réductase.

Le témoin positif, le finastéride, testé à 10μM, diminue de façon importante la métabolisation de la testostérone en DHT et inhibe donc l'activité de la 5-α réductase.

En ce qui concerne le 2,3-dihydroxypropyl dodécanoate, on observe également une inhibition de l'activité de l'enzyme 5-α réductase. De plus, on constate un effet-dose de cette inhibition : pour 75μM de 2,3-dihydroxypropyl dodécanoate l'activité de l'enzyme 5-α réductase est réduite à 66%.

Ces résultats démontrent que le 2,3-dihydroxypropyl dodécanoate inhibe bien l'activité de l'enzyme 5-α réductase.

Un objet de l'invention est donc l'utilisation du 2,3-dihydroxypropyl dodécanoate pour le traitement de la séborrhée, préférentiellement la séborrhée de la peau ou du cuir chevelu. La présente invention concerne l'utilisation du 2,3-dihydroxypropyl dodécanoate comme agent cosmétique sébomatifiant.

La séborrhée est souvent associée à l'alopécie androgénétique. L'alopécie se définit comme une perte de cheveux ou de poils, partielle ou totale. La vie d'un cheveu est soumise à un cycle dit cycle pilaire au cours duquel trois phases se succèdent. La phase anagène, c'est une période de croissance active et continue, associée avec une intense activité métabolique au niveau du bulbe. La phase catagène au cours de laquelle, les activités mitotiques sont ralenties. Le cheveu subit une involution, le follicule s'atrophie et son implantation dermique apparaît de plus en plus haute. La dernière phase est la phase télogène qui correspond à une période de repos du follicule et le cheveu fini par tomber poussé par un nouveau cheveu. Le cycle pilaire est achevé, un autre peut débuter. Il y a environ 20 à 25 cycles par vie de bulbes chez l'homme. Au cours du vieillissement les cheveux deviennent plus fins et leurs cycles plus courts.

Ainsi, l'alopécie androgénique héréditaire (que l'on appelait avant alopécie séborrhéique) est l'alopécie la plus fréquente et touche environ 70 % des hommes. Un cheveu dont le cycle de vie s'accélère est la première caractéristique des alopécies androgénétiques. Tous les autres symptômes ne sont que des conséquences de cette accélération. Au départ, le trouble de cette alopécie vient de ce que les androgènes hâtent le rythme de la phase anagène, forçant le cheveu à passer trop vite en phase télogène et ne laissant pas au follicule pileux assez de temps pour fabriquer une kératine de qualité. Un cercle vicieux s'installe alors : plus la fabrication du cheveu est rapide, plus celui-ci tombe vite et plus les cycles s'enchaînent rapidement, produisant à chaque fois un cheveu encore plus chétif et court que le précédent. A terme, le capital des cycles de renouvellement est épuisé, le follicule ne fabrique plus rien et meurt. Cette alopécie due à un excès d'androgènes touche également les femmes au moment de la ménopause ou à la suite d'un traitement par des androgènes. Elle commence au niveau des tempes et à la couronne.

Par conséquent, réduire l'activité de l'enzyme 5-α réductase devrait inhiber la séborrhée et de ce fait ralentir la chute des cheveux.

4) Composition

La présente invention concerne également les compositions cosmétiques comprenant du 2, 3-dihydropropyl dodécanoate en tant que seul principe actif pour son utilisation dans le traitement de la séborrhée. En particulier, la présente invention concerne les compositions cosmétiques comprenant du 2,3-dihydropropyl dodécanoate pour le traitement de la séborrhée de la peau ou du cuir chevelu. L'objet de l'invention correspond également à une méthode de traitement cosmétique de la séborrhée comprenant l'application sur la peau ou sur le cuir chevelu d'une composition cosmétique selon la présente invention. Il peut s'agir notamment d'une méthode de traitement cosmétique de la séborrhée de la peau ou du cuir chevelu.

Selon l'invention la composition cosmétique peut comprendre de 0,01 à 20% et de préférence de 0,5 à 10% et plus particulièrement de 1 à 5% en poids par rapport au poids total de la composition de 2,3-dihydroxypropyl dodécanoate.

Selon un autre mode de réalisation de l'invention, la composition cosmétique est celle comprenant en outre de l'eau thermale d'Avène.

La composition de l'eau thermale d'Avène est la suivante :

| Composition Eau Thermale d'Avène | mg/ml |
|---|---|
| Chlorures | 5,4 |
| Bicarbonates | 226,7 |
| Sulfates | 13,1 |
| Silice | 14 |
| Calcium | 42,7 |

(suite)

| Composition Eau Thermale d'Avène | mg/ml |
|---|---|
| Magnésium | 21,2 |
| Sodium | 4,8 |
| Potassium | 0,8 |
| Fer | 0,005 |
| Sélénium | 0,005 |
| Zinc | 0,02 |
| Cuivre | 0,005 |
| Résidu sec | 207 |
| Minéralisé | Faible |
| pH | 7,5 |
| Osmolarité | Hypotonique |

La présente description divulgue aussi une composition cosmétique dans laquelle le 2,3-dihydroxypropyl dodécanoate est le seul principe actif.

Selon l'invention, la composition cosmétique est celle comprenant du 2,3-dihydroxypropyl dodécanoate, de l'eau thermale d'Avène, et un support cosmétiquement acceptable.

Par « support cosmétiquement acceptable », on entend tout adjuvant ou excipient permettant la fabrication, la conservation ou l'administration de la composition cosmétique.

La composition selon l'invention peut se présenter sous toutes les formes normalement utilisées pour une application topique, notamment sous forme hydroalcoolique, d'une émulsion huile-dans-eau ou eau-dans-huile ou multiple, d'un gel huileux, ou d'un produit anhydre liquide, pâteux ou solide ou sous forme de dispersion en présence de sphérules. Ces compositions sont préparées selon les méthodes usuelles.

La composition peut se présenter sous toutes les formes galéniques envisageables. La composition peut avoir la forme d'une solution aqueuse, alcoolique, hydroalcoolique ou huileuse, d'une dispersion du type lotion ou sérum, d'une suspension, de microcapsules ou microparticules ; de dispersions vésiculaires de type ionique et/ou non ionique, d'une lotion aqueuse, huileuse ou sous forme de sérum ; d'une mousse, d'une préparation solide par exemple de stick ; d'une composition pour aérosol comprenant également un agent propulseur sous pression, d'un gel, ou sous forme de patch.

Cette composition peut être plus ou moins fluide et avoir l'aspect d'une crème blanche ou colorée, d'une pommade, d'un lait, ou d'une pâte. Elle peut éventuellement être appliquée sur la peau ou les cheveux sous forme d'aérosol.

La composition selon l'invention peut se présenter sous la forme d'une composition pour soins capillaires, notamment un shampoing, une lotion de mise en plis, une lotion, une crème ou gel coiffant, une composition de teinture, ou un gel antichute.

Elle peut également se présenter sous la forme d'une composition de nettoyage, de protection ou de soin pour le visage, ou pour le corps (par exemple crème de jour, crème de nuit, crème démaquillante, laits corporels de protection ou de soins, lotion, gel ou mousse pour le soin de la peau), une composition de maquillage telle que le fond de teint.

Lorsque la composition est une émulsion la proportion de la phase grasse peut aller de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine de la cosmétique.

- Les phases grasses :

Par phase grasse, on entend les composés lipophiles comme les huiles, les gommes, les pâtes et les cires.

Les huiles sont choisies de préférence parmi les huiles végétales, les huiles animales, les huiles minérales, les huiles de synthèse, les huiles siliconées, les esters d'acides gras liquides, les acides gras liquides et les amides gras liquides.

Comme huile végétale, on peut notamment mentionner l'huile d'amande douce, l'huile d'avocat, l'huile de ricin,

l'huile d'olive, la cire liquide jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de noyau d'abricot et l'huile de calophyllum.

Comme huile animale, on peut notamment citer le perhydrosqualène.

Comme huile de synthèse, on peut citer le squalane, les poly($\alpha$-oléfines) comme l'isododécane ou l'isohexadécane, les huiles végétales transestérifiées et les huiles fluorées.

Comme huiles siliconées, on peut citer les polydiméthylsiloxanes cycliques (nom INCI : cyclométhicone) tels que le décaméthyl pentasiloxane et les polyméthylsiloxanes linéaires de faible viscosité.

Les cires utilisables dans la présente invention sont par exemple les cires d'origine animale, végétale, minérale ou synthétique telle que la cire d'abeille, le spermaceti, les cires fluorées ou perfluorées, les cires de lanoline, les cires de Candellila, d'Ouricury, de Carnauba, du Japon, de beurre de cacao, les cires de fibres de liège ou de canne à sucre, la cire de son de riz, la cire de sapin, la cire de coton ; les cires microcristallines, la cire de paraffine, le petrolatum, la vaseline, l'ozokérite, la cire de montan ; les huiles hydrogénées ayant une température supérieure à 40°C comme l'huile de jojoba hydrogénée, les cires de polyéthylène et les cires obtenues par synthèse de Fischer-Tropsch.

Les cires peuvent également être choisies parmi les alcools gras cireux et esters gras cireux.

Les esters d'acides gras cireux sont des esters d'acides gras, c'est à dire des esters d'acides carboxyliques comportant au moins 10 atomes de carbone et d'un monoalcool ou d'un polyol. Les esters d'acides gras cireux utilisables dans la composition selon l'invention peuvent être des mono, des di, ou des triesters. A titre d'exemples d'esters cireux, on peut citer le myristate de myristyle et le stéarate de stérayle.

Les acides gras cireux utilisables dans la composition selon l'invention comportent de préférence de 12 à 24 atomes de carbone et peuvent être saturés ou insaturés, ramifiés ou non, et comporter une ou plusieurs fonctions hydroxy. On peut citer par exemple, l'acide laurique, l'acide stéarylique, l'acide cétylique et l'acide béhénique.

A titre d'amides cireux, on peut mentionner les céramides comme par exemple la noléyldihydrosphingosine.

- Les émulsionnants :

Les émulsionnants permettent de faciliter la dispersion de deux phases insolubles l'une par rapport à l'autre. L'émulsionnant et le coémulsionnant sont présents, dans la composition en une proportion allant de 0,3% à 30% et de préférence de 0,5 à 20% et préférentiellement de 0,5 à 10% en poids par rapport au poids total de la composition.

Le système émulsionnant peut comprendre en particulier un ou plusieurs composés choisis parmi les alcools gras éthoxylés, les esters d'acides gras et de PEG, les glycérides partiels d'acides gras éthoxylés, les triglycérides d'acide gras polyglycérolés et leurs dérivés éthoxylés.

On peut citer en tant qu'alcools gras éthoxylés selon l'invention, les produits d'addition d'oxyde d'éthylène avec l'alcool béhénylique, notamment ceux comportant de 6 à 12 groupes oxyéthylénés (par exemple Beheneth-9 ou Beheneth-10) ; les produits d'additions d'oxyde d'éthylène avec l'alcool stéarylique, notamment ceux comportant 6 à 12 groupes oxyéthylénés (par exemple steareth-9) ; les produits d'addition d'oxyde d'éthylène avec l'alcool isostéarylique, par exemple ceux comportant 6 à 12 groupes oxyéthylénés (Isosteareth-9), et leurs mélanges.

On peut également citer en tant que tensio-actifs non ioniques, des alcools gras oxyéthylénés différents de ceux décrits précédemment, à savoir les produits d'addition d'oxyde d'éthylène avec l'alcool laurylique (laureth-9 à laureth-50) ; les produits d'addition d'oxyde d'éthylène avec l'alcool cétéarylique ou cétylstéarylique (Ceteareth-9 à Ceteareth-30), les produits d'addition d'oxyde d'éthylène avec l'alcool cétylique (Ceteth-9 à Ceteth-30) ; et leurs mélanges.

Des tensioactifs additionnels peuvent également être compris dans la composition. Ces tensioactifs additionnels peuvent être les sels d'acide gras ayant 8 à 30 atomes de carbones comme par exemple les sels d'acide palmitique, l'acide stéarique, l'acide béhénique, les esters gras de glycérol, comme par exemple le glycéryl stéarate ; les dérivés oxyéthylénés des sels d'acides gras et des esters gras de glycérol comportant 2 à 8 groupes d'oxyde d'éthylène et leurs mélanges, ainsi que tout émulsionnant et agent de conditionnement connu de l'homme du métier.

- Les conservateurs :

Les conservateurs sont présents dans la composition en une proportion allant de 0,1% à 5% et de préférence de 0,1% à 1% en poids par rapport au poids total de la composition.

La composition peut comprendre en outre des agents antimicrobiens tels que des conservateurs ou agents antifongiques choisis parmi les alcools, pouvant contenir un ou plusieurs substituants aromatiques, par exemple les phénoxyéthanol comme le 2-phénoxyéthanol, 1-phénoxy-2-propanol, l'alcool benzylique, le 2-hydroxybiphényl, les parabènes, comme le méthylparabène, éthylparabène, propylparabène, butylparabène, isobutylparabène, méthylparabène de sodium, éthylparabène de sodium, propylparabène de sodium, isobutylparabène de sodium, butylparabène de sodium ou isobutylparabène de sodium, l'urée de l'imidazolidinyle, l'urée du diazolidinyle, l'hydroxyméthylglycinate de sodium,

des dérivés halogénés tels que butylcarbamate d'iodopropynyle, le 2-bromo-2-nitropropan-1,3-diol, 2,4,4'-trichloro-2'-hydroxydiphényléther (triclosan), le 3,4,4'-trichlorocarbanilide (triclocarban), le chlorbutanulum, l'alcool 2,4-dichlorobenzylique, l'urée du N-(4-chlorphényl-N'-(3,4-dichlorphényle, le 1,2-dibromo-2,4-dicyanobutane, le chloroxylénol, le cétoconazole, l'oxiconazole, le butoconazole, le clotrimazole, l'éconazole, l'énilconazole, le fenticonazole, le miconazole, le sulconazole, le tioconazole, le fluconazole, l'itraconazole, le terconazole, des actifs contenant un ou plusieurs azotes cationiques tels que le chlorure de cétyltriméthylammonium, le chlorure de cétylpyridinium, le chlorure de benzéthonium, le chlorure de diisobutyléthoxyéthyl-diméthylbenzylammonium, le chlorure de diisobutyl-phénoxy-éthoxyéthyl-diméthylbenzyl-ammonium, le chlorure, bromure, saccharinate de N-alkyl-N,N-diméthyl-benzyl-ammonium, le chlorure de triméthylammonium, l'aluminiumchlorohydroxylacétate de sodium, le chlorure de tricétylméthylammonium, diaminoalkylamide, des acides organiques et leurs sels, tel que l'acide citrique, des agents antimicrobiens insaturés tels que le farnesol, la terbinafine ou la naftifine, des agents aromatiques hétérocycliques tels que le bifonazole, le cloconazole, l'isoconazole, de tout autre agent antimicrobien ou antifongique connu de l'homme du métier; et leurs mélanges.

- Les épaississants :

Les épaississants sont présents dans la composition en une proportion allant de 0,1% à 20% et de préférence de 0,1% à 10% en poids par rapport au poids total de la composition.

La composition peut également comprendre des agents épaississant ou des agents de modification de la rhéologie, tels que par exemple des uréthanes nonioniques éthoxylés hydrophobiquement modifiés, des acides polycarboxyliques épaississants tels que le copolymère d'acrylates/steareth-20 méthacrylate, des carbomères, des copolymères d'acrylates et acrylates $C_{10}$-$C_{30}$ alkyl acrylate réticulés ; les gommes naturelles et les argiles, les argiles modifiées comme les bentones, les sels métalliques d'acide gras comme les stéarates d'aluminium et la silice hydrophobe ; et leurs mélanges.

- Les ajusteurs de pH :

La composition peut également comprendre des acides et des bases permettant d'ajuster la zone de pH de ladite composition. Les bases peuvent être minérales (soude, potasse, ammoniaque) ou organiques telles que la mono-, di- ou triéthanolamine, un aminométhylpropanediol, la N-méthyl-glucamine, les acides aminés basiques comme l'arginine et la lysine ; et leurs mélanges.

La composition peut en outre comprendre un ou plusieurs autres ingrédients tels que des tampons de pH, des vitamines, des fragrances, et tout autre composé utile bien connu de l'homme du métier.

Les exemples suivants illustrent de façon non limitative la présente invention.

Exemple 1 : Composition pour la peau

| Ingrédients (INCI désignation) | Pourcentage |
|---|---|
| Eau | QSP 100 % |
| Glycérine | 3 |
| Phénoxyéthanol | 0,35 |
| $Na_2EDTA$ | 0,1 |
| Polyacrylate-13 et Polyisobutène et Polysorbate 20 & eau | 1 |
| Stéarate de glycéryle et PEG-100 Stéarate | 4 |
| Alcool cétylique | 1 |
| Cyclopentasiloxane | 5 |
| Glycérol tri-2-éthylhexanoate | 3 |
| Carbonate de dicapryle | 2 |
| 2,3-dihydroxypropyl dodécanoate | 2 |
| Chlorphénésine | 0,27 |
| Polyméthylacrylate | 2 |
| Parfum | 0,1 |

Exemple 2 : Composition pour les cheveux

| | |
|---|---|
| 2,3-dhydroxypropyl dodécanoate | 1% |
| Lauryl éther sulfate de sodium | 9% |
| Lauryl bétaïne | 2% |
| Alkyléther sulfate de sodium magnésium | 1% |
| PEG-18 Glycéryl oléate/cocoate | 1% |
| Polyquaternium 22 | 0,5 % |
| NaCl | qsp visco |
| Acide citrique | qsp pH |
| eau | qsp 100 gr |

Exemple 3 : Composition pour les cheveux

| | |
|---|---|
| 2,3-dihydroxypropyl dodécanoate | 0,5 % |
| Lauryl éther sulfate de sodium | 9% |
| Chlorure de cétrimonium | 0,3 % |
| Tween 20 | 5% |
| $Na_2EDTA$ | 0,2 % |
| Epaississant | 4% |
| NaCl | qsp visco |
| Acide citrique | qsp pH |
| eau | qsp 100 gr |

ABREGE DESCRIPTIF

La présente invention concerne l'utilisation du 2,3-dihydroxypropyl dodécanoate pour le traitement de la séborrhée.

## Revendications

1. Le 2,3-dihydroxypropyl dodécanoate pour son utilisation en tant que seul principe actif pour le traitement de la séborrhée dans une composition cosmétique.

2. Le 2,3-dihydroxypropyl dodécanoate, pour son utilisation selon la revendication 1, **caractérisé en ce que** la composition cosmétique se présente sous la forme d'une composition capillaire.

3. Le 2,3-dihydroxypropyl dodécanoate pour son utilisation selon la revendication 1, **caractérisé en ce que** la composition cosmétique se présente sous la forme d'une composition de nettoyage, de protection ou de soin pour le visage, ou pour le corps, en particulier une crème de jour, crème de nuit, crème démaquillante, un lait corporel de protection ou de soin, une lotion, un gel ou une mousse pour le soin de la peau, ou encore d'une composition de maquillage telle que le fond de teint.

4. Le 2,3-dihydroxypropyl dodécanoate pour son utilisation selon la revendication 1, **caractérisé en ce que** la séborrhée est celle du cuir chevelu.

5. Le 2,3-dihydroxypropyl dodécanoate pour son utilisation selon la revendication 1, pour ralentir la chute des cheveux.

6. Le 2,3-dihydroxypropyl dodécanoate pour son utilisation selon la revendication 1, **caractérisé en ce que** la séborrhée est celle de la peau

7. Le 2,3-dihydroxypropyl dodécanoate pour son utilisation selon la revendication 1, **caractérisé en ce que** la composition comprend de l'eau thermale d'Avène..

**Patentansprüche**

1. 2,3-Dihydroxypropyldodecanoat zu dessen Verwendung als einziger Wirkstoff zur Behandlung von Seborrhö in einer kosmetischen Zusammensetzung.

2. 2,3-Dihydroxypropyldodecanoat zu dessen Verwendung nach Anspruch 1, **dadurch gekennzeichnet, dass** die kosmetische Zusammensetzung die Form einer Haarbehandlungszusammensetzung aufweist.

3. 2,3-Dihydroxypropyldodecanoat zu dessen Verwendung nach Anspruch 1, **dadurch gekennzeichnet, dass** die kosmetische Zusammensetzung die Form einer Reinigungs-, Schutz- oder Pflegezusammensetzung für das Gesicht oder für den Körper, insbesondere einer Tagescreme, einer Nachtcreme, einer Abschminkcreme, einer Bodylotion zum Schutz oder zur Pflege, einer Lotion, eines Gels oder eines Schaums zur Hautpflege, oder auch einer Schminkzusammensetzung wie Make-up aufweist.

4. 2,3-Dihydroxypropyldodecanoat zu dessen Verwendung nach Anspruch 1, **dadurch gekennzeichnet, dass** die Seborrhö die Kopfhaut betrifft.

5. 2,3-Dihydroxypropyldodecanoat zu dessen Verwendung nach Anspruch 1 zum Abschwächen von Haarausfall.

6. 2,3-Dihydroxypropyldodecanoat zu dessen Verwendung nach Anspruch 1, **dadurch gekennzeichnet, dass** die Seborrhö die Haut betrifft.

7. 2,3-Dihydroxypropyldodecanoat zu dessen Verwendung nach Anspruch 1, **dadurch gekennzeichnet, dass** die Zusammensetzung Avene-Thermalwasser umfasst.

**Claims**

1. Use of 2,3-dihydroxypropyl dodecanoate as a single active ingredient for treating seborrhoea in a cosmetic composition.

2. Use of 2,3-dihydroxypropyl dodecanoate according to claim 1, **characterised in that** the cosmetic composition is in the form of a hair treatment composition.

3. Use of 2,3-dihydroxypropyl dodecanoate according to claim 1, **characterised in that** the cosmetic composition has the form of a cleansing, protection or facial care composition, or for the body, in particular a day cream, night cream, make-up removal cream, protective or treating body lotions, skin care lotion, gel or foam, or a make-up composition such as foundation composition.

4. Use of 2,3-dihydroxypropyl dodecanoate according to claim 1, **characterised in that** the seborrhoea is that of the scalp.

5. Use of 2,3-dihydroxypropyl dodecanoate according to claim 1, to slow down hair loss.

6. Use of 2,3-dihydroxypropyl dodecanoate according to claim 1, **characterised in that** the seborrhoea is that of the skin.

7. Use of 2,3-dihydroxypropyl dodecanoate according to claim 1, **characterised in that** the composition comprises Avene thermal spring water.

# Figure 1

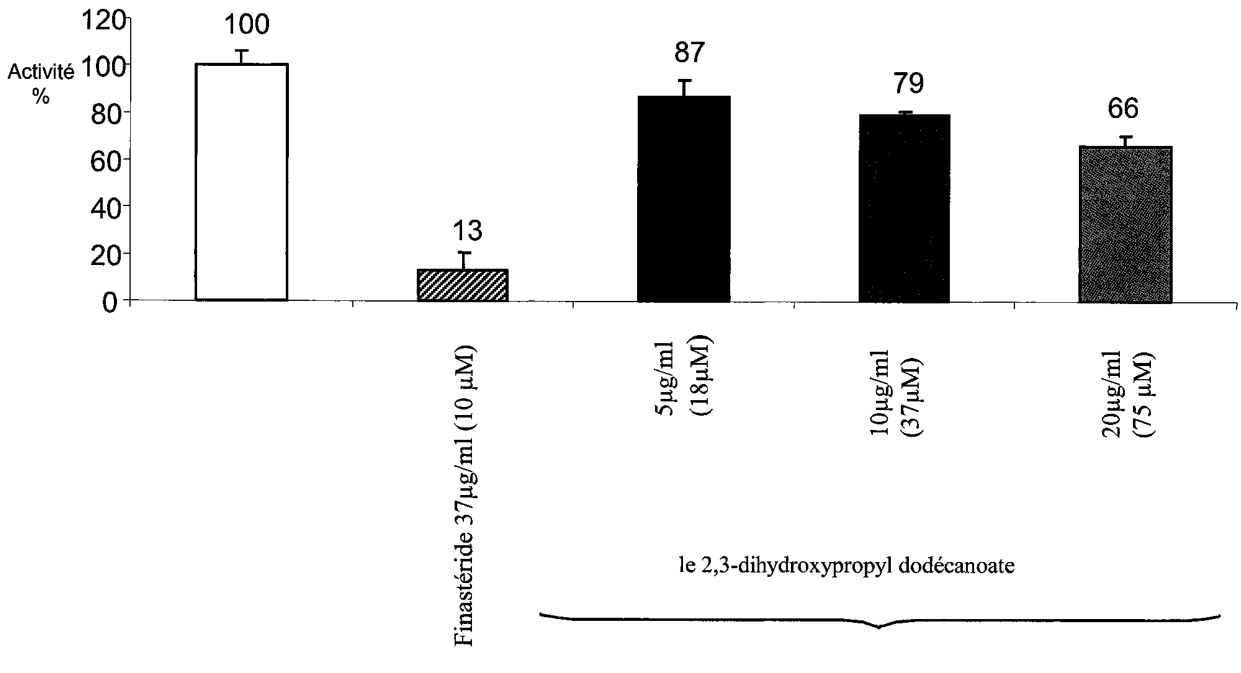

Activité %
120
100
80
60
40
20
0
100
13
87
79
66
Finastéride 37µg/ml (10 µM)
5µg/ml (18µM)
10µg/ml (37µM)
20µg/ml (75 µM)
le 2,3-dihydroxypropyl dodécanoate

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2009203628 A


### Littérature non-brevet citée dans la description


- **LUU-THE et al.** Characterization, Expression and Immunohistochemical localization of 5-alpha reductase in human skin. *The Journal of Investigate Dermatology,* 1994, 221-226